(19) Europäisches Patentamt
European Patent Office
Office européen des brevets

(11) **EP 4 654 158 A1**

(12) **EUROPEAN PATENT APPLICATION**

(43) Date of publication:
26.11.2025 Bulletin 2025/48

(21) Application number: 25173629.4

(22) Date of filing: 30.04.2025

(51) International Patent Classification (IPC):
*G06V 20/69* (2022.01)     *G01N 33/543* (2006.01)
*G06T 7/00* (2017.01)

(52) Cooperative Patent Classification (CPC):
G06V 20/69; G01N 33/54313; G06T 7/0012

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 20.05.2024 SE 2430277

(71) Applicant: MABTECH AB
131 28 Nacka Strand (SE)

(72) Inventors:
• **Smedman, Christian**
SE-131 28 Nacka Strand (SE)
• **Jaldén, Joakim**
SE-131 28 Nacka Strand (SE)
• **Del Aguila Pla, Pol**
SE-131 28 Nacka Strand (SE)
• **Magnusson, Klas**
SE-131 28 Nacka Strand (SE)

(74) Representative: **Basck Limited**
**9 Hills Road**
**Cambridge CB2 1GE (GB)**

(54) **IMPROVED IMMUNOASSAY**

(57)     A method for determining positions of microspheres in an image of an immunoassay utilizing microspheres, wherein the image comprises a plurality of depictions of microspheres (110), wherein the method is characterized in that the method comprises determining the positions of microspheres by determining a distribution (P) based on a deconvolution of an observed luminescence ($O_d$) and a mathematical representation (b) of a microsphere (bead) as observed by a microscope, wherein the distribution (P) provides the likelihood that there is a microsphere (110) at a given position in the image (140).

EP 4 654 158 A1

510 RECEIVE LUMINESCENCE

515 PROVIDE ILLUMINATION MAP

520 DETERMINE INITIAL DISTRIBUTION

530 GENERATE PREDICTION

540 UPDATE

550 DETERMINE DISTANCE

560 COMPARE

562 DISTANCE LARGE ITERATIONS LEFT

561 DISTANCE SMALL ITERATIONS DONE

570 DETERMINE FINAL DISTRIBUTION

580 DETERMINE BEAD POSITIONS

FIG. 5

**Description**

TECHNICAL FIELD

The teachings herein relate to immunoassays utilizing microspheres and in particular immunoassays that enable an improved localization of microspheres.

BACKGROUND

An immunoassay is a biochemical test that measures the presence or concentration of a biomarker through the use of a capture molecule attached to a suitable surface. For different assays the capture molecule can either be an antibody or antigen, and the suitable surface is typically a high-binding plastic, different forms of membranes or microspheres (microspheres, hereafter also referred to as beads) containing appropriate chemical groups that allow for antibody or antigen conjugation. The molecule detected by the immunoassay is referred to herein as an analyte and is in many cases a protein, such as cytokines or hormones, although it may also be antigen-specific antibodies.

Detection and quantification of analytes involve a secondary reagent where a detectable signal is produced, typically via fluorescence emission from a detection complex that is bound to the captured analyte in a sandwich complex. The intensity of the signal is proportional to the concentration of the analyte in the sample and many times involves a two-step detection process with a biotinylated detection antibody in combination with a streptavidin fluorophore conjugate. Analytes in biological liquids, such as serum, plasma or cell supernatants, are frequently measured using immunoassays for medical and research purposes.

Microspheres are commonly used as a suitable surface for the chemical attachment of capture molecules due to their production simplicity and the potential to chemically dye microspheres into distinguishable subsets using different concentrations of suitable fluorophores (subset-identity-dye). The dye, or mixture of dyes, is permanently captured within the microsphere and enables elegant multiplexing in immunoassays. The microspheres are in some cases magnetic, thereby facilitating all wash steps necessary in bead-based immunoassays. Once a sandwich complex has been established, microspheres are washed and typically analyzed on flow cytometers where each bead is passed through a flow cell one-by-one and excited by multiple lasers. The emission from the detection complex is determined using one laser while any fluorescent dye captured within the microsphere is identified using (an)other laser(s).

While flow-based assays are standard in the analysis of microspheres, they also come with some major drawbacks. One disadvantage is their overall complexity that requires yearly maintenance in order to keep lasers aligned, but most importantly, microsphere samples will inherently contaminate the instrument with potentially dangerous pathogens and can eventually clog the cytometer.

The prior art thus suffers from many drawbacks.

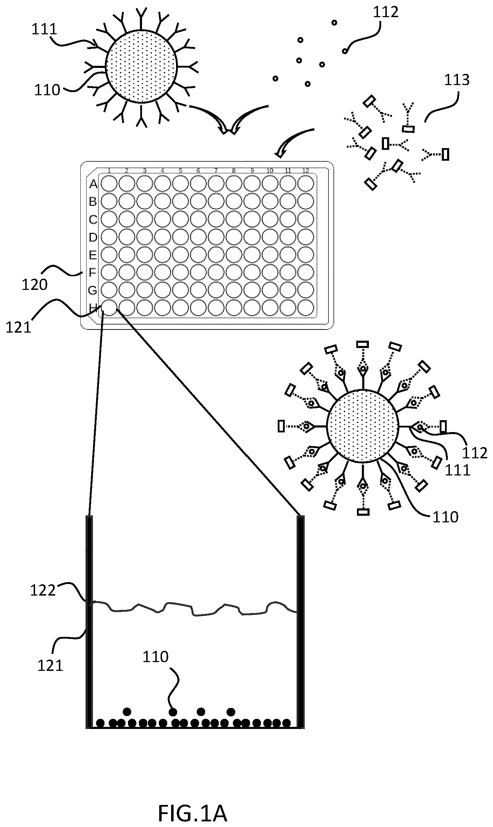

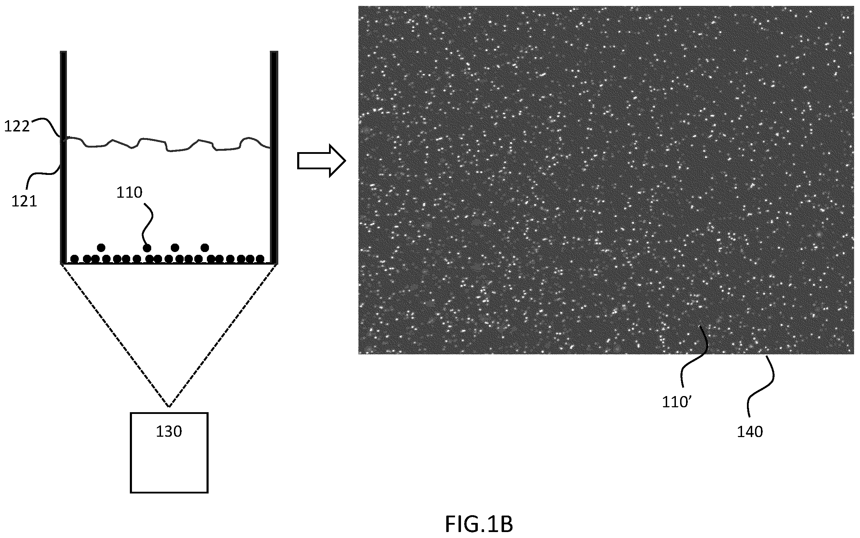

The general procedure of an immunoassay utilizing beads is shown schematically in figure 1A and 1B, where a plurality of microspheres, hereafter referred to as beads 110 are in one out of several wells 121 in an assay plate 120 that can be for example in a 96-well or 384-well format. The beads 110 that belong to different subset-identities are coated with different capture molecules also referred to as binding agents 111, such as antibodies or antigens depending on the usage. The analytes 112 are introduced to the beads 110 and following addition of detection antibodies 113 the analytes 112 are bound in 111 in a sandwich complex.

Figure 1A also shows a sideview of a well 121 where a surface level of the test solution 122 is indicated (wavy form exaggerated to indicate that it is a fluid). Also shown in this figure is how a majority of the beads 110 have been allowed to sediment to the bottom of the well before being analyzed.

By having different subsets of fluorescently dyed beads 110 coated with different capture molecules 111 (binding different analytes 112) in the same well 121, a test for several analytes may be done in a single well 121, thereby saving both time, assay plates, and the test solution.

As the analytes 112 have bound to the beads 110 (through the binding agents 111), and possibly after other procedural steps such as washing, an image 140 is captured by a scanning microscope or other imaging device 130 using various fluorescent wavelengths (for example 488nm, 532nm, 638 nm, 642 nm, 640 nm provided by multiple lasers and/or multiple emission filters - as is known in the art), wherein the image has depictions 110' of beads 110, as shown in figure 1B. The image 140 is then to be analyzed in order to determine the number of beads, their individual intensity and most importantly their correct bead position, as for example indicated by the bead-centers, present in the test solution. It should be noted that although determining the bead centers is discussed herein, this is only one way of determining a location for a bead, and other alternatives are possible and are treated as equivalents herein. Although in some specific embodiments it is the bead centers that are determined. In figure 1B, the imaging device 130 is arranged under the well 121, however, the imaging device 130 may be placed differently with regards to the well 121, such as for example above. The placement of the imaging device 130 may be dependent on which type of imaging device 130 is used.

As the beads 110 have been allowed to sediment to the bottom of well, they will be randomly dispersed (or distributed) in the image.

These beads (or microspheres) 110 all have a bead-center (and thus also a bead-center location) in the fluorescent channels relating to subset-identity-dye(s) and a co-localized analyte signal in another fluorescent channel. However, due to various factors such as beads aggregating and forming randomized clusters, the image 140 is often difficult to analyze, wherefor it can be (very) difficult to determine the correct center-point for each bead 110. This is necessary in order to avoid incorrect readouts when determining their microsphere-subset-identity and their co-localized analyte signal.

Figure 1B shows an enlarged view of a subsection of the image in figure 1A, where (depictions of) beads 110 are clearly visible. As noted herein, the beads 110 and their bead-centers 110 are sometimes difficult to identify in clusters, and some beads may be of a low intensity and therefore difficult to perceive in a complex multiplex assay as some beads 110 will cluster and may occlude or mask bead-centers of lower intensity.

To enable an accurate analysis - both quantitative and qualitative - it is important that the beads 110, and in many cases the bead centers, are correctly identified or located in the image 140.

The prior art shows various manners of improving the quality of the image 140 through different image processing techniques in order to improve the image 140 so that the bead-centers corresponding to the beads 110 are more easily recognizable. However, such prior art image processing is bound by problems of the image and cannot satisfactorily handle all situations.

SUMMARY

As noted above, the prior art suffers from many drawbacks and the inventors have therefore realized that there is a need to establish new technologies for analyzing microspheres in a more reasonable manner. The inventors are therefore proposing an image-based approach for analyzing dyed microspheres through the transparent bottom of a common multi-well plate (for example in a 96-well or 384-well format). This results in a no-contact readout of microspheres that elegantly solves many of the issues associated with flow-cytometers as described above.

In order for an image-based approach to be successful, bead locations (such as bead-centers) of randomly distributed microspheres must be accurately identified in order to determine their correct microsphere-subset-identity and their co-localized analyte signal. This is achieved by using a new form of mathematical analysis of a multi-channel fluorescence microscopy image, for which both the excitation light and the filtering of the emitted light have been chosen to image a specific fluorophore.

The inventors have realized - after insightful and inventive reasoning - an improved manner of identifying - or locating - the centers of beads in an image, which overcomes several drawbacks of the prior art techniques.

The improved manner which will be discussed herein is provided through a method for a method for determining positions of microspheres in an image of an immunoassay utilizing microspheres, wherein the image comprises a plurality of depictions of microspheres (110), wherein the method is characterized in that the method comprises determining the positions of microspheres by determining a distribution ($P$) based on a deconvolution of an observed luminescence ($O_d$) and a mathematical representation ($b$) of a microsphere (bead) as observed by a microscope, wherein the distribution ($P$) provides the likelihood that there is a microsphere (110) at a given position in the image (140) as per the appended claims.

It should be noted that the description herein makes references to microspheres, and that this is to be understood to be commonly known particles used in immunoassays, which are also known as microspheres, particles, microparticles, or beads. The terminology thus does not refer to the common definition of a sphere in mathematics.

The description herein provides for method for determining positions of microspheres in an image of an immunoassay utilizing microspheres utilizing a deconvolution is made based on the mathematical model to yield a probability distribution indicating the likelihood that there was a bead center located at a particular location at the time of imaging.

A "blob" (to use the language of the prior art patent application published as US2014120556A1) is of an indistinct shape and such indistinct blobs are difficult to differentiate from one another when they are side by side or partially overlapping.

A simple filtering based on intensity - as is proposed by US2014120556A1 - will not be able to differentiate between such "blobs".

In contrast to the prior art being based on convolution, the present invention is based on a deconvolution.

As is known, a convolution operates with known quantities, and in the case of for example US2014120556A1 or US2012268584A1 basically provides edge detection. Whereas a deconvolution attempts to return to an original image, i.e. the opposite of convolution.

In the present invention, where it is unknown if there are one, two or more overlapping particles giving rise to the same "blob" it is not possible to do a straightforward deconvolution of a convolution based on a shape (the convolution of US2014120556A1) as this will not be able to differentiate between one or more particles.

The present invention therefore proposes a brilliant solution in that a deconvolution is made to yield a probability that there is a center of a particle at a given point at the time of imaging. The present invention thus makes a series of guesses that there was a particle at given points and sees how such guesses align with the actual reality. Clearly not the same as filtering an image based on intensity levels as in US2014120556A1.

In some embodiments the method is characterized in that the method further comprises determining the positions of microspheres by receiving the observed luminescence ($O_d$), determining an initial distribution ($P_n$), providing a prediction for luminescence ($L_n$) based on the initial distribution ($P_n$), updating the distribution ($P_{n+1}$) utilizing an update rule ($U$), determining a distance ($d$) between the prediction for luminescence ($L_n$) and the observed luminescence ($O_d$), wherein the update rule is designed to reduce the distance ($d$), for a predetermined number of iterations or until the distance ($d$) is below an acceptance threshold level, and if so determining a final distribution ($P$) as the distribution ($P_{n+1}$).

In some embodiments the method further comprises, if the distance ($d$) is not below an acceptance threshold level, providing an updated prediction for luminescence ($L_{n+1}$) based on the updated distribution ($P_{n+1}$), determining a distance ($d$) between the updated prediction for luminescence ($L_{n+1}$) and the observed luminescence ($O_d$), comparing the updated prediction for luminescence ($L_n$) to the observed luminescence ($O_d$) by determining if the distance ($d$) is below the acceptance threshold level.

By utilizing a mathematical model comprising a representation of the physical characteristics of a microsphere and a distribution on the image as discussed herein it is possible to not only identify the beads - or the location of the beads - but also to identify individual beads within clusters of beads, even when the beads have different light intensities. It is equally possible to locate the centers of the beads. The teachings herein can equally be used to locate the beads, and then to determine the centers of the beads, as well as be used to locate the centers of the beads directly, by simply adapting the distribution to be the of a center at any given point. For the purpose of the teachings herein, these two approaches will be discussed simultaneously without much differentiation, as a skilled person would be able to differentiate the two and know how to adapt one to the other.

It should be noted that this does not represent an improvement of the image, even if improvements of the image may be applied to improve the results, but a mathematical approach for determining the positions - mathematically - for microspheres based on a model comprising a mapping, based on a realistic representation of a microsphere, between a hypothesized bead center distribution and the observable image.

According to one aspect there is also provided a computer program product comprising program instructions for performing the method according to any preceding claim when executed by one or more processors in a processing device.

According to one aspect there is also provided a processing device comprising a memory and a processing unit, wherein the processing device is configured to configured to determine positions of microspheres in an image of an immunoassay utilizing microspheres, wherein the image comprises a plurality of spots, by determining the positions of microspheres by determining a distribution ($P$) based on a deconvolution of an observed luminescence ($O_d$) and a mathematical representation ($b$) of a microsphere as observed by a microscope, wherein the distribution ($P$) indicates the likelihood that there is a microsphere at a given position in the image.

It should be noted that when reference is given to an image it is to be understood to include the digital representation of an image, i.e., the image data. The image data may in some embodiments be stored in a format provided by the imaging device, for example as image sensor data. The image data may in some embodiments be stored in a format with low or minimal refining, such as compression or filtering. The image data may in some embodiments be stored in a RAW format. The image data may in some embodiments be stored in any known or unknown image format.

BRIEF DESCRIPTION OF FIGURES

The teachings herein will be discussed with reference to the attached figures where

Figure 1A shows a schematic view of an immunoassay system,
Figure 1B shows how an image to be analyzed is provided in an immunoassay system,
Figure 2 shows a schematic view of a processing system according to the teachings herein,
Figure 3A shows a schematic view of a microsphere representation according to the teachings herein,
Figure 3B shows a schematic view of a microsphere representation according to the teachings herein,
Figure 3C shows a schematic view of a distribution according to the teachings herein,
Figure 4 shows a schematic view of an algorithm according to the teachings herein,
Figure 5 shows a flowchart for a general method according to the teachings herein, and
Figure 6 shows a schematic view of a computer-readable medium carrying computer instructions according to the teachings herein.

DETAILED DESCRIPTION

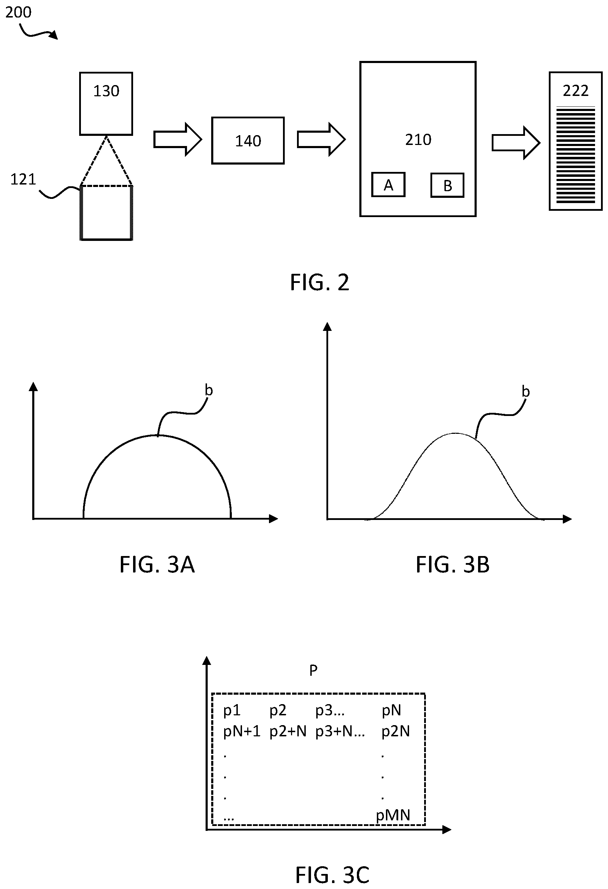

Figure 2 shows a schematic view of a processing system 200 arranged to determine locations of microspheres also referred to as beads 110 in an image 140 (showing a plurality of depictions 110 of beads 110) as in figure 1A. As noted with reference to figure 1A, no difference will be made between the bead and the depiction of the bead herein.

The processing system 200 comprises a processing device 210. The processing device 210 comprises one or more processing units (A), which are configured to control partial or overall functionality of the processing system 200. The processing device 210 comprises (or is connected to) a memory B carrying computer-readable instructions that when loaded into at least one of the one or more processing units enables the processing device 210 to carry out the teachings herein. As a skilled person would understand, the one or more processing units may comprise, but are not limited to, a microcontroller, a microprocessor, a central processing unit (CPU), a graphics processing unit (GPU), a complex instruction set computing (CISC) processor, an application-specific integrated circuit (ASIC) processor, a Field-Programmable Gate Array (FPGA), a reduced instruction set (RISC) processor, a very long instruction word (VLIW) processor, and other processors or control circuitry. As a skilled person would also understand, the memory is a computer-readable storage medium and examples of implementations of computer-readable storage medium include, but are not limited to, Electrically Erasable Programmable Read-Only Memory (EEPROM), Random Access Memory (RAM), Read Only Memory (ROM), Hard Disk Drive (HDD), Flash memory, a Secure Digital (SD) card, Solid-State Drive (SSD), and/or CPU cache memory.

The processing device 210 receives an image 140 of a well 121 in an assay plate 120 that has been captured by a scanning device or other imaging device 130. In some embodiments, the scanning device 130 is comprised in the processing system 200. In some embodiments, the scanning device 130 is external to the processing system 200 and the processing system receives the image 140 - directly or indirectly - from the imaging device 130.

In some embodiments, the imaging device 130 is a digital camera. In some embodiments, the imaging device 130 is a microscope equipped with a digital camera.

In some embodiments, the imaging device 130 is a confocal microscope. Confocal microscopy, most frequently confocal laser scanning microscopy (CLSM) or laser scanning confocal microscopy (LSCM), is an optical imaging technique for high optical resolution and contrast of a micrograph by means of using a spatial pinhole to block out-of-focus light in image formation. A confocal microscope focuses a beam of light at one narrow depth level at a time. The CLSM achieves a controlled and highly limited depth of focus, where out-of-focus light is screened out.

As is discussed in relation to figure 1B, the image 140 shows randomly distributed beads 110 that sometimes overlap. The beads may also be diffuse or difficult to perceive. In cases where the beads are magnetic, the beads will also have an increased tendency to cluster. This makes the problem of determining which objects in the image 140 correspond to beads a difficult problem as discussed briefly in relation to figure 1B. The problem may also be defined as determining the locations of beads. In some embodiments, the location of the bead to be determined is the center of the bead. The output of the system 200 is a list (or other representation) 222 of bead locations (xy coordinates) in the image. In some embodiments, the output 222 also comprises the intensity of the bead(s), for example the peak (pixel) intensity or an integrated reception of emission from the bead(s). In some embodiments, the output 222 also comprises other properties of the beads, such as other mathematically derived features or characteristics, such as focus depth. The output 222 may be provided as an image, a mathematical representation, as a modified version of the image 140 or as a table.

In order to solve this problem, the inventors are proposing - instead of simply applying various image processing techniques - to represent the beads with a mathematical representation (of physical properties of the beads, in some embodiments as observed by a microscope), and then to apply an algorithm, that will be discussed in detail in the below, to fit an observation model being a mapping of a mathematical representation of a bead to the depictions of beads in the image 140. The inventors are thus proposing to utilize a model-based source point localization algorithm (hereafter referred to as the algorithm), where a source point corresponds to (a center of) a bead. The algorithm is based on three main concepts, namely

　　a realistic physical observation model $M,$
　　an inverse problem formulation and
　　an optimization method.

The realistic physical observation model $M$ has two parts. The first part (i.e. the mathematical representation discussed in the above) is designed to represent a bead, the bead being a source point for a depiction in the image 140. In some embodiments, the mathematical representation is therefore designed so as to model or represent how a bead would be observed by a microscope. In some embodiments, a bead could thus be represented as a sphere.

It should be noted that even though the detailed description herein is focused on a confocal microscope, the teachings can equally be applied to other types of microscopes.

As the inventors have further realized, the observation of the bead is dependent on the microscope being used, and a bead being observed using a confocal microscope would not be observed as a sphere - as the confocal microscope only provides focus in a plane (or close to a plane). However, as the inventors have also further realized, the observation of the bead is not dependent as such on the bead itself, but on the fluorophores in the bead and more precisely, the luminescence of the fluorophores. The bead observed will thus be observed as being denser in the middle. The inventors have therefore - after inventive and insightful reasoning - designed a mathematical representation based on a half-sphere to represent the bead.

Figure 3A shows a schematic view of one example of a mathematical representation (or function) $b$ ($b$ for bead), being (proportional to) a half-sphere. It should be noted that whereas figure 3A (and figure 3B) shows the mathematical representation as a function of one variable (i.e., the $x$-axis), the mathematical representation is in fact a function of two variables representing $xy$-coordinates the image. In some embodiments the half-sphere is (half-)circular. In some embodiments the half-sphere is (half-)round, round to be taken to not be perfectly circular.

As the inventors have further realized, the observation in this case is the observation being made by the processor receiving the images from the microscope, and as the inventors have realized, the microscope adds optical (or other) distortions to the image observed and, as the inventors have realized, these distortions should therefore be taken into account when designing the representation for the bead.

And as the inventors have realized, such distortions may be modeled as a convolution between the point-spread function of the microscope, or an approximation of the point-spread function, and the mathematical representation (half-sphere or other shape) of the bead. In some embodiments the (approximation of) the point-spread function may be modelled as an airy disk or a Gaussian kernel in some embodiments the mathematical representation $b$ of the half-sphere thus also includes (a Gaussian, Airy or other) blur. Figure 3B shows a schematic view of one example of a mathematical representation $b$ being a half-sphere with added Gaussian blur.

The second part of the realistic physical observation model (hereafter also the physical model $M$) is designed to model the observations as a function of possible (hypothesized) bead centers. The physical model $M$ is, in some embodiments, a convolution of a (bead center) distribution $P$ with a kernel $b$, where the kernel $b$ is the mathematical representation of (physical characteristics of) the beads 110 as discussed above. The inventors have - after insightful and inventive reasoning - realized that the mathematical representation of a bead can be used as a kernel for a deconvolution of the observation in order to yield a density function indicating the of a bead being in any given point, thus providing a solution to the inverse problem mentioned above as will be discussed in detail in the below. Figure 3C shows a schematic view of such a distribution $P$. The distribution $P$ gives $p_i$ that represents a likelihood that a bead exists at a position $i$, and in the example of figure 3C the positions are arranged in a rectangle. The rectangle may in some embodiments correspond to the image, wherein each position $i$ corresponds to a pixel, a group of pixels, or a point on a sub-pixel grid. In some embodiments each position $i$ corresponds to a portion of the image having the size of a bead. In some embodiments, each position $i$ corresponds to a portion of a scan of the image, wherein the portion may have the size of a bead or smaller. A portion having a smaller size enables for handling a distribution of beads where the possible positions are not uniformly placed.

It should be noted that the teachings herein do not only deconvolve with the point-spread-function, PSF, of the microscope, but with the entire bead model. The deconvolution of the teachings herein is thus fundamentally different from established deconvolution practices. It should also be noted that it is the (precise) model as disclosed herein of the bead that allows us to discard out of focus (floating) beads and other non-bead artefacts.

In the figure, an indication is given of the image by the dotted lines indicating a rectangle representing the image 140 of figure 1A.

It should be noted that even though the distribution $P$ is given in vectorized format (one index) where each row is handled in sequence herein, other formats are also possible, for example a 2-dimensional (matrix) notation may be used where a first index represents a row, and a second index represents a column. It should also be noted that the numbering used may also change and any numbering starting for example at 0 or at 1 may be used.

As is well-known to a skilled person, namely a person being highly skilled in mathematics, a collection of observed data $O_d$ may be expressed as a function $M$ (hereafter referred to as the forward operator or simply model) applied to a distribution (hereafter $P$), i.e. $M(P) = O_d$. The forward problem is then simply to find $O_d$ given $P$, i.e., to determine $M(P)$.

As discussed in the above, the inventors have realized that by setting the observed data ($O_d$) to be the image of the beads, a range of previously established algorithms for solving inverse problems may be utilized to give the solution in terms of $P$.

Thus, in the situation where the observed data ($O_d$) is set to be the image of the beads 110, the inverse problem is to find $P$ given $O_d$ being the observed beads 110 of the image 140, wherein the inventors are proposing to use $M$ as discussed in the above, namely a deconvolution of observed data to yield the distribution $P$ in the case that $M$ is a convolution, or that $P$ is the solution to an inverse problem in general.

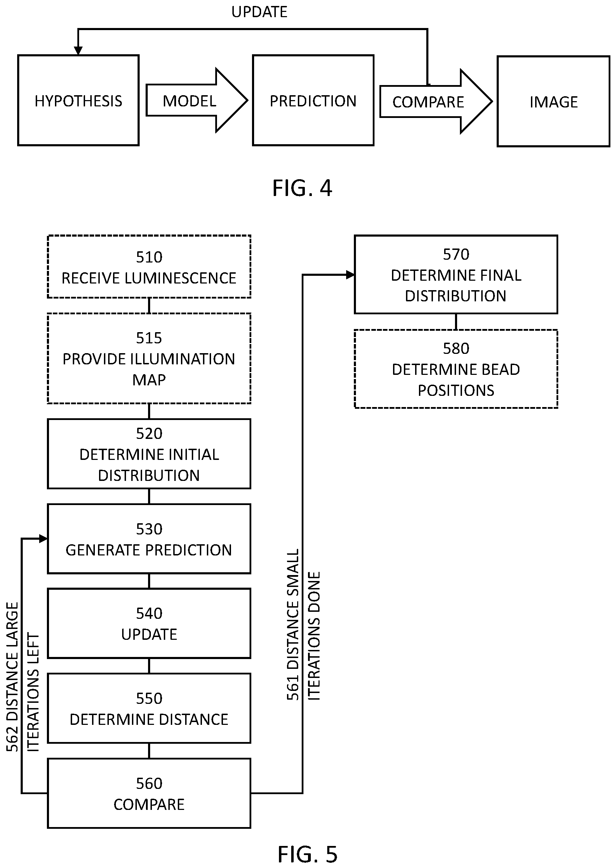

In order to solve this inverse problem, the inventors are proposing a mathematical model $M$ to be used in an iterative algorithm as per figure 4, where the mathematical model $M$ is applied to a hypothesis resulting in a prediction which is compared to the observed data. If the prediction does not match the observed data, an update of the hypothesis is done and the model $M$ is (again) applied to the updated hypothesis. This is repeated until the (updated) prediction corresponds to the observation.

The starting hypothesis is in some embodiments that beads (or analytes) are distributed uniformly over the well. And, in this case - when the prediction corresponds to the observation - the value at position $i$ is proportional to the likelihood that the bead exists in that position $i$. The distribution then gives the locations of the beads in the image 140, the location of the bead(s) being the position(s) having a "high" value.

In the below, one solution out of many will be discussed in more detail. As a skilled person would understand, there are many options for solving such an inverse problem and given that the observation is modelled using a bead center density function $P$ paired with a representation $M$ of the mapping of bead positions to observations made by the microscope, the work to provide such a solution is straightforward.

In this example, the algorithm employed is based on a (proximal) gradient algorithm. Other embodiments may utilize other algorithms known to solve inverse problems, such as a sparsity promoting regularization, alternating direction method of multipliers (ADMM), multiplicative update rules, and Forward-Backward splitting algorithms. For example, sparsity-promoting regularization may be implemented in form of $L_1$-regularization or group-sparsity regularization based on (non-squared) form of $L_2$-regularization over the image. In another example, multiplicative update rules and/or Forward-Backward splitting algorithms may facilitate optimization of variables during such deconvolution. Examples of proximal gradient algorithms include, but are not limited to, iterative shrinkage thresholding algorithms (ISTA), multiplicative update rules, and the accelerated proximal gradient algorithms (FISTA).

As a skilled person would realize, the (proximal) gradient algorithm is an example of a deconvolution algorithm that can be used to provide the solution. A skilled person would understand how to provide the solution based on another known algorithm, especially after having read the details presented herein, which a skilled person would understand to adapt and modify in a routine manner for a different algorithm.

It should be noted that as a bead is observed by the fluorophores emitting light (after being excited by an excitation light) the distribution $P$ actually becomes a quantity proportional to the amount of emitted light that would be causally induced in the observation due to a bead centered at the given point $i$.

The update rule used when updating the hypothesis depends on the algorithm chosen. As a skilled person would understand, many alternatives exist, which are all well-known to a skilled person. Using for example (proximal) gradient-based update rules, the update rule which updates the distribution for each point would be based on the difference (or other comparative metrics) between the prediction and the observation. And if the prediction is that there is a higher luminescence at a given point than actually observed, the luminescence at that point is reduced accordingly.

Figure 5A shows a flowchart for a general method according to the teachings herein, an observed luminescence is received 510. In some embodiments the observed luminescence is received as an image of a well 140. The image 140 may be received from the microscope and processed by a processor of the microscope, the microscope then also being a processing device 210. The image 140 may be received externally to the microscope and processed by a processor of a processing device 210 outside the microscope. Examples of such a processing device 210 may be a processing device 210 connected to the microscope or a processing device 210 receiving the image via a storage medium and/or a connection (such as an internet connection) enabling for later or remote processing. The image shows various beads 110. As noted, and discussed above, the image may be received in any format. In some embodiments, the image is received in a RAW format or a format having no or little preprocessing performed as is also discussed in the above. Receiving the image in such a format provides for a more accurate determination of the beads as the formatting may otherwise filter out subtle indications in the image.

The image 140 is then optionally processed to provide 515 an illumination map, where the luminescence of each point (pixel, group of pixels or other point as discussed above) is noted. The illumination map will be noted as $O_d$ in the below representing a vector (or matrix) of the luminescence $L$ at any point $i$ in the observed image $O_d$. Optionally the image is utilized as the illumination map $O_d$. The illumination map is thus one possible representation of the observed luminescence $O_d$.

Referring to figure 4, the hypothesis is stated 520. In some embodiments the hypothesis is set to be that the distribution is uniform (same value) for all points $i$. The value is in some embodiments determined based on the analyte to be researched, where an analyte generally being easy to bind or find will have a higher (initial) value than an analyte which is generally difficult to bind or find. In some embodiments, the value is set to for example 0 or 1.

Thereafter a prediction 530 is generated based on this hypothesis, wherein the prediction, in some embodiments, predicts a luminescence in each point $i$; $L_n = M(P_n)$, where $M$ is the observation model above, being a mapping between the distribution $P$ (vector or matrix of (distribution) values $p_i$ due to possible beads at point $i$) at iteration $n$ and the luminescence $L$ (vector or matrix of luminescence $l_i$ at point $i$) at iteration $n$.

It should be noted that capitals are used hereafter to represent a vector (or matrix), where $P$ is the vector of values $p$.

An update rule $U$ is applied to the prediction in order to update 540 the distribution $P$, i.e., to update $P_n$ to $P_{n+1}$, wherein $P_{n+1} = U(P_n, L_n, O_d)$, where $O_d$ is the observed luminescence as mentioned above. The update rule $U$ is designed to operate so that the difference or distance $d$ between the observed luminescence $O_d$ and the predicted luminescence $L_n$

is reduced. This may be expressed as $d(L_{n+1}, O_d) < d(L_n, O_d)$. As noted above, the update rule depends on the algorithm chosen. The scientific optimization literature provides a plethora of update rules $U$ that guarantee convergence, i.e., monotonicity of the sequence $d(L_n, O_d)$ in $n$. The theory that guarantees this is readily available in the scientific literature, and although stringent proofs of monotonicity are technically difficult, the update operators are relatively simple.

A distance $d$ is thus also determined 550. In some embodiments the distance $d$ is determined based on the observed luminescence as captured in the image, $O_d$. In some embodiments the distance $d$ is determined as the square (Euclidean) distance between predicted luminescence $l_i$ and the observed luminescence $o_i$ summed up over all points $i$; $d = \Sigma_i(l_i - o_i)^2$.

Then a determination or comparison 560 is made whether the distance $d$ is sufficiently small 561. If the distance is not sufficiently small 562, a new hypothesis is generated 530 based on the updated distribution; $L_{n+1} = M(P_{n+1})$, a new update is performed 540 and a new distance is determined 550. The distance $d$ is sufficiently small if the distance $d$ is below an acceptance threshold level.

If the distance is sufficiently small 561, the final distribution is determined 570 and is given by $P = P_{n+1}$ and the (final) distribution $P$ is utilized to locate 580 bead positions.

In some embodiments the comparison 560 is made regarding a predetermined repetitions and while the predetermined repetitions have not been made, the procedure continues 561, and when the repetitions have been made 562, the final distribution is determined 570 and is given by $P = P_{n+1}$.

As discussed in the above, the bead positions are given by the points having a "high" distribution value. In some embodiments the bead positions are determined in later processing perhaps in another processing device and the actual determination 580 is thus optional as the final model is the main result of the algorithm herein. This is indicated by the dotted box in figure 5. Similarly, the actual reception of the image and determination of the illumination map is optional in that it may be performed by another processing device as the only input needed is the illumination map $O_d$. This is also indicated by the dotted box in figure 5.

The determination 560 whether the distance $d$ is sufficiently small is in some embodiments made by comparing the distance $d$ to a threshold distance value. The threshold distance value can be chosen in many different ways as a skilled person would realize, including set by an operator in order to investigate different scenarios.

In some embodiments, the determination that the distance is below an acceptance threshold level is made based on the number of iterations, where the distance is assumed to be sufficiently small after a given number of iterations, for example 40, 80, 120 or any range therein between.

In some embodiments, the determination that the distance is below an acceptance threshold level is made based on the change in $L_n$ and when the change in $L_n$ (i.e., $|L_{n+1} - L_n|$) is below a threshold level, the distance is deemed to be sufficiently small.

As noted above, a skilled person would be able to formulate or implement an algorithm as per the teachings herein based only on the information given in relation to the flowchart of figure 5, but in order to facilitate an understanding also by persons other than skilled persons, a more detailed description will also be given.

The teachings herein thus utilize a mathematical (functional) relation or model $M$ that maps a (typically) two-dimensional (relative) distribution $P$ representing possible bead centers to an expected fluorescent image $L$ for a given fluorophore, i.e., $L = M(P)$, given that particular $P$. The algorithm obtains a measured illumination $O_d$ or illumination map, possibly from an image sensor of (such as a camera in or connected to) a microscope and operates to recover or determine a final distribution $P$ such that $L = M(P)$ is as close to $O_d$ as possible in some sense, measures by a distance function $d(L, O_d)$.

In some embodiments, the distance measured is the sum of squared differences between the predicted illuminance $L_n$ and the measured illuminance $O_d$, summed over all pixel positions. Finding the distribution $P$ that satisfies this property is typically hard to do in one step, and an iterative approach is used instead. To this end:

510. The illuminance image 140 is obtained, the image representing the observed data $O_d$, possibly from the camera of a microscope.

520. An initial hypothesized (typically uniform) model $P_0$ is created. Let $n = 0$.

530. The current model $P_n$ is used to predict a luminescence $L_n = M(P_n)$.

540. An update rule $U$ is used to update $P_n$ to $P_{n+1}$, where

$$P_{n+1} = U(P_n, L_n, O_d)$$

550. A distance $d$ is also determined and the update is performed so that $d(L_{n+1}, O_d) < d(L_n, O_d)$

560. This is repeated until $d(L_{n+1}, O_d)$ is sufficiently small or a predetermined number of iterations has been reached. The repetition is affected by letting $n = n + 1$ and repeating at 530.

570. If $d(L_{n+1}, O_d)$ is sufficiently small or a predetermined number of iterations has been reached, then present $P = P_{n+1}$

as the final model, and proceed to
580. use $P$ to identify possible bead positions.

As mentioned above, different update rules may be used, and in some embodiments, what is referred to as a (proximal) gradient-based update rule for $P$. In embodiments where the mapping $M$ is linear, which means that we can write $L = AP$, where $A$ is a matrix of suitable dimensions and where $P$ and $L$ are vectorized representations of the model and the expected luminescence, respectively. For the squared difference distance metric d, where

$$d(L, O_d) = \|L - O_d\|^2 = \|AP - O_d\|^2$$

and where $\|\cdot\|^2$ is the squared Euclidean norm, the update rule takes the form

$$P_{n+1} = [P_n - \eta A^T(AP_n - O_d)]_+$$

where $A^T$ is the transpose of $A$, where $\eta$ is an appropriately chosen step length, and where $[\cdot]_+$ denotes element-wise positivity.

Alternative update rules follow from the use of proximal optimization and operator splitting and have slightly more complicated forms.

As discussed in the above and with reference to figure 3A, the representation of a bead as seen by the microscope may take the form proportional to a half-sphere. Mathematically this may be expressed as below. Assuming a bead of radius $r$ located at the center of a coordinate system. Mathematically, a coordinate is inside a bead if the three-dimensional coordinate ($x, y, z$) satisfies $x^2 + y^2 + z^2 \leq r^2$, and outside the bead if not.

The mathematical representation $B(x, y, z)$ of where a (centered) bead is found in some embodiments becomes:

$$B = 1 \texttt{ if } x^2 + y^2 + z^2 \leq r^2$$

$$B = 0 \texttt{ if } x^2 + y^2 + z^2 > r^2$$

The mathematical representation $B(x, y, z)$ is thus an indicator function indicating where the bead is in three spatial dimensions, from which the intensity profile $b$ observed by the microscope can be derived. The intensity profile $b$ is thus a mathematical representation of a microsphere as observed by a microscope.

The intensity profile $b$ of the observation of the bead in a sample is, in some embodiments, proportional to a half-sphere mass $H = H(x, y)$ indicating the thickness of the bead at location ($x, y$). Note that this may not be the same as the intensity profile of the bead in the image, as, for example, the PSF of the optics or the luminosity of the fluorescence has not been considered. In the $xy$-plane, the number $\gamma$ of photons emitted per unit area and time is rather given by $\gamma =$

$$2\alpha\sqrt{r^2 - x^2 - y^2} = 2\alpha H(x, y)$$
, where $\alpha$ is a proportionality constant accounting for the number of photons emitted per bead volume and time. Figure 3A shows a schematic illustration of a $b$ proportional to $H$ showing a non-distorted (including impurities) observation of the bead(s).

As discussed in the above and below and with reference to figure 3B, the intensity profile, $b$, of a bead, $B$, in the image may be computed in some embodiments by adding Gaussian blur by assuming a Gaussian approximation of the point-spread function of the microscope.

It should be noted that this derivation of $b$ is also only one example, and other representations may also or alternatively be used. In some embodiments intensity profile $b$ of the observation of the bead is based on empirical observation data.

Below a mathematical derived representation of a bead that takes into account characteristics of the microscope distortions will be discussed.

The mathematical representation of the microscope point spread function (PSF) $f(x, y, z)$ is in some embodiments approximated by a Gaussian as

$$f(x, y, z) = q_0 e^{-\frac{1}{2\sigma_x^2}(x - x_m)^2 - \frac{1}{2\sigma_y^2}(y - y_m)^2 - \frac{1}{2\sigma_z^2}z^2}$$

where $\sigma_x = \sigma_y \ll \sigma_z$, where $\sigma_x, \sigma_y, \sigma_z$ are the focal distance in the $x, y$, and $z$ direction, respectively, where $z$ is the direction of the microscope (i.e. the point of view for the microscope), where $x_m$ and $y_m$ are the microscope focus position in the $xy$-

plane, and where $q_0 = (2\pi)^{-\frac{3}{2}}(\sigma_x\sigma_y\sigma_z)^{-1}$ is the well-known normalizing constant of the three-dimensional multivariate Gaussian distribution.

This representation of the physics assumes that beads have a constant concentration of fluorophores inside the three-dimensional bead, and that the focus distance in the third dimension (perpendicular to the image plane) is very long in relation to the resolution in the imaging plane. Under the assumptions made, the luminosity $L$ originating from a single bead positioned at the origin of the $xy$-plane and picked up by the microscope when focused on position $(x_m, y_m)$ of the $xy$-plane becomes

$$L(x_m, y_m) = \int B(x,y,z)f(x,y,z)dxdydz = \int \left(\int B(x,y,z)f(x,y,z)dz\right)dxdy$$

As the inventors have realized, it may be assumed that $\sigma_z \gg r$ (i.e., that the focus distance is long in relation to the bead radius) the inner integral becomes

$$\int B(x,y,z)f(x,y,z)dk = q_0 e^{-\frac{1}{2\sigma_i^2}(x-x_m)^2 - \frac{1}{2\sigma_j^2}(y-y_m)^2} \int R(x,y,z)e^{-\frac{1}{2\sigma_z^2}z^2} dz$$

$$\approx q_0 e^{-\frac{1}{2\sigma_x^2}(x-x_m)^2 - \frac{1}{2\sigma_y^2}(y-y_m)^2} q_1 H(x,y)$$

where $H(x, y)$ is the half-sphere mass given by

$$H(x,y) = \begin{cases} \sqrt{r^2 - x^2 - y^2} & x^2 + y^2 \leq r^2 \\ 0 & x^2 + y^2 > r^2 \end{cases}$$

and where $q_1 = 2(2\pi)^{1/2}\sigma_z$ is another scaling constant accounting for all constant factors. The half-sphere $H(x, y)$ is used herein - as the inventors have realized is sufficient - only to represent a quantity proportional to (half) the amount of fluorophores along a (focusing) line in the $z$-dimension through the bead.

In embodiments where the amount of fluorophores is not uniform throughout the sphere, or if fluorophores deep inside the sphere are masked by the surrounding sphere, the function $B(x, y, z)$, and hence $H(x, y)$, can be replaced by a more representative measure of the contribution made by the fluorophores in an position $(x, y, z)$ in the bead to the luminosity seen by the microscope.

Also, the function $f(x, y, z)$ can be replaced in such cases by another approximation for the point-spread function (PSF) of the microscope.

Proceeding with the assumptions made (uniform and non-masked fluorophores), the luminosity profile $L(x_m, y_m)$ at microscope (focus) position $(x_m, y_m)$ from a single bead becomes

$$L(x_m, y_m) \approx$$

$$q_0 q_1 \int e^{-\frac{1}{2\sigma_x^2}(x-y_m)^2 - \frac{1}{2\sigma_y^2}(y-y_m)^2} H(x,y)dxdy = q_0 q_1 \int e^{-\frac{1}{2\sigma_x^2}(x_m-x)^2 - \frac{1}{2\sigma_y^2}(y_m-y)^2} H(x,y)dxdy \triangleq$$

$$q_0 q_1 e^{-\frac{1}{2\sigma_x^2}x_m^2 - \frac{1}{2\sigma_y^2}y_m^2} * H(x_m, y_m).$$

where * denotes the convolution operator.

In other words, the intensity observed from a sphere centered at the origin is proportional to the convolution of a Gaussian and a half-sphere. Using linearity and translation invariance, we get the expected luminosity of all beads (probabilistically) positioned according to the distribution $P(x, y)$ according to

$$L(x,y) = b(x,y) * P(x,y) \Leftrightarrow L = M(P)$$

where, as depicted in figure 3B,

$$b(x,y) \propto e^{-\frac{1}{2\sigma_x^2}x^2 - \frac{1}{2\sigma_y^2}y^2} * H(x,y)$$

In other words, the intensity profile $b$ is utilized as a kernel in the convolution.

In some embodiments this has to be discretized (as opposed to being continuous) to adapt it for use in a processing device.

To summarize, the representation of the expected luminosity $L$ is given by the convolution of $P$ with a (bead) kernel $b$. Thus, $M$ is a convolution and the inverse problem of obtaining $P$ from the observed image $O_d$ becomes a deconvolution in such embodiments.

The kernel $b$ depends on the representation of the bead at the origin of the $xy$-plane and in particular the radius $r$ of the bead and the assumption of a uniform fluorophore distribution throughout the bead.

Since the convolution operator $M$ is linear we can write the relation $L = M(P)$ as $L = AP$ using vector representations of $P$ and $L$ as described before.

In some embodiments $AP$ or $A^T P$ are implemented as linear (convolution) operators (as opposed to as explicit matrix vector multiplications). This in order to speed up the processing as the convolution operators are less computationally demanding.

It should be noted that the teachings herein do not exclude the possibility of applying (various) image processing techniques to the image prior to performing the determination of the teachings as discussed herein.

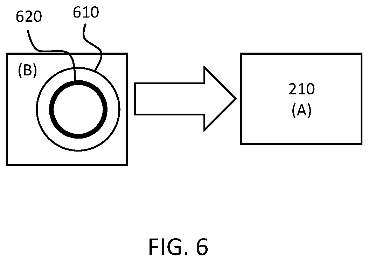

Figure 6 shows a computer program product 610 comprising program instructions 620 for performing the method and teachings as discussed herein when executed by one or more processors (A) in a processing device 210. The computer program product 610 is implemented as an algorithm, embedded in a software stored in the non-transitory computer-readable storage medium having program instructions stored thereon, the program instructions being executable by one or more processors in the computer system to execute the method and teachings herein. The non-transitory computer-readable storage means may include, but are not limited to, an electronic storage device, a magnetic storage device, an optical storage device, an electromagnetic storage device, a semiconductor storage device, or any suitable combination of the foregoing, such as the memory B of the processing device 210. Examples of implementation of computer-readable storage medium, but are not limited to, Electrically Erasable Programmable Read-Only Memory (EEPROM), Random Access Memory (RAM), Read Only Memory (ROM), Hard Disk Drive (HDD), Flash memory, a Secure Digital (SD) card, Solid-State Drive (SSD), a computer-readable storage medium, and/or CPU cache memory.

**Claims**

1. A method for determining positions of microspheres in an image of an immunoassay utilizing microspheres, wherein the image comprises a plurality of depictions of microspheres (110), wherein the method is **characterized in that** the method comprises

   determining the positions of microspheres by
   determining a distribution ($P$) based on a deconvolution of an observed luminescence ($O_d$) and a mathematical representation ($b$) of a microsphere as observed by a microscope, wherein the distribution ($P$) provides the likelihood that there is a microsphere (110) at a given position in the image (140).

2. The method according to claim 1, wherein the method is further **characterized in that** the deconvolution of the observed luminescence ($O_d$) is based on a model ($M$), where $M$ is a convolution of the distribution ($P$) with a kernel ($b$), where the kernel ($b$) is an intensity profile of the bead as observed by the microscope, wherein the kernel ($b$) includes a mathematical representation of optical characteristics of a microscope.

3. The method according to claim 2, wherein the method is further **characterized in that** the mathematical model (M) comprises a representation of the physical characteristics wherein the physical characteristics comprises a luminescence observed by an imaging device (130) for the microsphere (110).

4. The method according to any preceding claim, wherein the method is **characterized in that** the method further comprises determining the positions of microspheres by

   receiving (510) the observed luminescence ($O_d$),
   determining (520) an initial distribution ($P_0$),
   providing (530) a prediction for luminescence ($L_n$) based on the initial distribution ($P_0$),
   updating (540) the distribution ($P_{n+1}$) utilizing an update rule ($U$),

determining (550) a distance ($d$) between the prediction for luminescence ($L_n$) and the observed luminescence ($O_d$), wherein the update rule is designed to reduce the distance ($d$), for a predetermined number of iterations or until (560) the distance ($d$) is below an acceptance threshold level, and if so determining (570) a final distribution ($P$) as the distribution ($P_{n+1}$).

5. The method according to claim 4, wherein the method further comprises, if the distance ($d$) is not below an acceptance threshold level,

providing (530) an updated prediction for luminescence ($L_{n+1}$) based on the updated distribution ($P_{n+1}$), determining (550) a distance ($d$) between the updated prediction for luminescence ($L_{n+1}$) and the observed luminescence ($O_d$), comparing (560) the updated prediction for luminescence ($L_n$) to the observed luminescence ($O_d$) by determining if the distance ($d$) is below the acceptance threshold level.

6. The method according to any of claims 4 to 5, wherein the distribution $P$ indicates that there is a microsphere at a given position by indicating a center of a bead causally creating luminescence ($l_i$) at the given point ($i$).

7. The method according to any of claims 4 to 6, wherein the method further comprises determining (580) the location of microspheres based on the final distribution ($P$) through distribution values ($p_i$) at a point ($i$) giving the location of a microsphere at that point ($i$).

8. The method according to any of claims 4 to 7, wherein the method further comprises determining the initial distribution ($P_0$) as a uniform distribution, and/or wherein the update rule is based on a gradient-based algorithm, and/or wherein the update rule is a multiplicative update rule.

9. The method according to any preceding claim, wherein the method further comprises receiving the observed luminescence ($O_d$) by receiving the image.

10. The method according to any preceding claim, wherein at least some of the microspheres are magnetic microspheres, and/or wherein at least some of the microspheres are dyed with fluorophores, that when excited by an excitation light emit a luminescence, wherein the fluorophores are uniformly distributed in the microspheres.

11. The method according to any preceding claim, wherein the representation of the microsphere is given by a function proportional to a half-sphere.

12. The method according to claim 11, wherein the representation of the microsphere is given by a half-sphere with added Gaussian blurring.

13. The method according to any of claims 4 to 12, wherein the method further comprises determining the distance $d$ as the squared difference between predicted luminescence ($l_i$) and the observed luminescence ($o_i$) summed up for each point;

$$d = \sum_i (l_i - o_i)^2 .$$

14. A computer program product (610) comprising program instructions (620) for performing the method according to any preceding claim when executed by one or more processors (A) in a processing device (210).

15. A processing device (210) comprising a memory (R) and a processing unit (A), wherein the processing device (210) is configured to determine positions of microspheres in an image of an immunoassay utilizing microspheres, wherein the image comprises a plurality of spots, by

determining the positions of microspheres by determining a distribution ($P$) based on a deconvolution of an observed luminescence ($O_d$) and a mathematical representation ($b$) of a microsphere as observed by a microscope, wherein the distribution ($P$) indicates the that there is a microsphere (110) at a given position in the image (140).

FIG.1A

FIG.1B

EP 4 654 158 A1

200

FIG. 2

FIG. 3A

FIG. 3B

FIG. 3C

UPDATE

| HYPOTHESIS | MODEL ⟶ | PREDICTION | COMPARE ⟶ | IMAGE |

FIG. 4

510
RECEIVE LUMINESCENCE

515
PROVIDE ILLUMINATION
MAP

520
DETERMINE INITIAL
DISTRIBUTION

530
GENERATE PREDICTION

540
UPDATE

550
DETERMINE DISTANCE

560
COMPARE

562 DISTANCE LARGE
ITERATIONS LEFT

561 DISTANCE SMALL
ITERATIONS DONE

570
DETERMINE FINAL
DISTRIBUTION

580
DETERMINE BEAD
POSITIONS

FIG. 5

FIG. 6

EUROPEAN SEARCH REPORT

Application Number

EP 25 17 3629

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SARDER P. ET AL: "Estimating Locations of Quantum-Dot-Encoded Microparticles From Ultra-High Density 3-D Microarrays", IEEE TRANSACTIONS ON NANOBIOSCIENCE., vol. 7, no. 4, December 2008 (2008-12), pages 284-297, XP093287965, US ISSN: 1536-1241, DOI: 10.1109/TNB.2008.2011861 * the whole document * ----- | 1-15 | INV. G06V20/69 G01N33/543 G06T7/00 |
|  |  |  | TECHNICAL FIELDS SEARCHED (IPC) |
|  |  |  | G06V G06T G01N |

The present search report has been drawn up for all claims

| Place of search | Date of completion of the search | Examiner |
|---|---|---|
| The Hague | 18 June 2025 | Neubüser, Bernhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2014120556 A1

- US 2012268584 A1